# EUROPEAN PATENT APPLICATION

(11) **EP 4 610 252 A1**
(43) Date of publication of application: **03.09.2025**
(21) Application number: 23883112.7
(22) Date of filing: 26.10.2023
(51) Int. Cl.: C07C 333/20, A61K 31/27, A61P 11/00, A61P 43/00, A23L 33/13

(54) **NOVEL COMPOUND AND PHARMACEUTICAL COMPOSITION INCLUDING SAME AS ACTIVE INGREDIENT FOR PREVENTION OR TREATMENT OF PULMONARY FIBROSIS**

(30) Priority: 26.10.2022 KR 20220139548
(71) Applicant: Gil Medical Center, Incheon 21565 (KR); Gachon University of Industry-Academic Cooperation Foundation, Seongnam-si, Gyeonggi-do 13120 (KR)
(72) Inventor: JEONG, Sung Hwan, Incheon 21584 (KR); MAENG, Han-Joo, Incheon 22002 (KR); SEO, Seung-Yong, Incheon 22001 (KR); SON, Eun Suk, Incheon 22766 (KR)
(74) Representative: Sagittarius IP
(86) International application number: PCT/KR2023/016703
(87) International publication number: WO 2024/091007

(57) **Abstract**

The present invention relates to a novel compound and a pharmaceutical composition for preventing or treating pulmonary fibrosis, comprising the same as an active ingredient, specifically, a compound represented by Formula I below; a pharmaceutical composition for preventing or treating pulmonary fibrosis, comprising the compound as an active ingredient; method for preventing or treating pulmonary fibrosis using the pharmaceutical composition; and a food composition for preventing or improving pulmonary fibrosis, comprising the compound as an active ingredient. 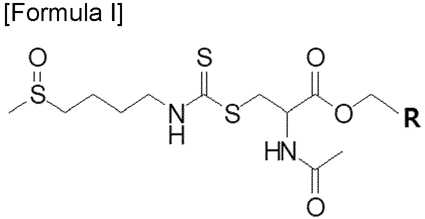 wherein, in Formula I above,
R is methyl or ethenyl.

## Description

### [Technical Field]

The present invention relates to a novel compound and a pharmaceutical composition for preventing or treating pulmonary fibrosis comprising the same as an active ingredient. Specifically, it relates to a compound represented by Formula I; a pharmaceutical composition for preventing or treating pulmonary fibrosis comprising the compound as an active ingredient; a method for preventing or treating pulmonary fibrosis using the pharmaceutical composition; and a food composition for preventing or treating pulmonary fibrosis comprising the compound as an active ingredient. 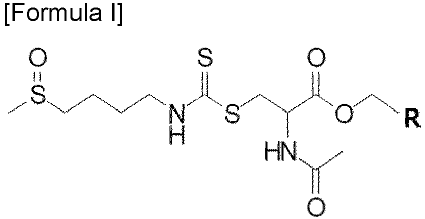 wherein, in Formula I above,
R is methyl or ethenyl.

### [Background Art]

Fibrosis refers to the formation of excessive fibrous connective tissue in organs or tissues during the regeneration process and the like, which contrasts to normal fibrous tissues formed in organs and tissues. Examples of fibrosis include pulmonary fibrosis, liver fibrosis, kidney fibrosis, pancreatic fibrosis, endomyocardial fibrosis, mediastinal fibrosis, myelofibrosis, retroperitoneal fibrosis, necrotic fibrosis (lung), nephrogenic systemic fibrosis (skin), Crohn's disease, keloids, myocardial infarction, systemic scleroderma, *etc.*

Among them, pulmonary fibrosis or idiopathic pulmonary fibrosis (IPF) is a representative lung disease in which repeated inflammation caused by alveolar damage induces fibrosis and leads to respiratory failure in patients. Among pulmonary fibrosis, IPF is a progressive disease for which the cause has not yet been identified and in which dyspnea and coughing worsen and lead to death by respiratory failure within three to four years of diagnosis, and the five-year survival rate is approximately 30% to 40%, which is similar to that of lung cancer.

The current treatment of pulmonary fibrosis mainly involves the use of steroids or immunosuppressants, which are cytotoxic drugs. Between steroids and the cytotoxic drugs, steroids are used first, and a therapy of a steroid in combination with azathioprine or cyclophosphamide is currently used as a treatment for radiation-induced pulmonary fibrosis (Ochoa *et al.,* Journal of Medical Case Reports, 6:413.2012).

Additionally, Esbriet (active ingredient: pirfenidone) from Roche and Ofev (active ingredient: nintedanib) from Boehringer Ingelheim are known as drugs for treating or improving pulmonary fibrosis. Among these drugs, pirfenidone, which received FDA approval in 2014, is known to mainly inhibit the action of TGF-β, thereby delaying the worsening and progression of IPF as an anti-inflammatory and anti-fibrotic agent, while nintedanib exhibits an anti-fibrotic effect as a multiple tyrosine kinase inhibitor.

However, both drugs show limited therapeutic effects as treatments for early or moderate IPF and can cause gastrointestinal side effects such as diarrhea, abdominal pain, loss of appetite, and impaired liver function, and photosensitivity. Since these drugs only alleviate the decrease in lung function and do not exhibit a fundamental therapeutic effect, there is a need to develop more effective treatments for pulmonary fibrosis.

### [Disclosure]

### [Technical Problem]

As a result of conducting research in an effort to develop a candidate substance capable of treating and improving pulmonary fibrosis more effectively than existing substances, the present inventors confirmed that a novel sulforaphane-based compound exhibits an inhibitory effect on pulmonary fibrosis by regulating the expression of genes and proteins related to pulmonary fibrosis and completed the present invention.

### [Technical Solution]

An object of the present invention is to provide a compound represented by Formula I below or a pharmaceutically acceptable salt thereof.

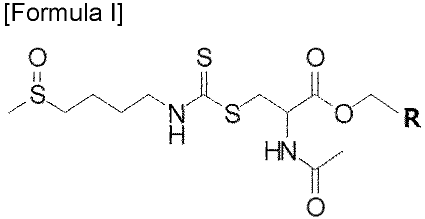

Another object of the present invention is to provide a pharmaceutical composition for preventing or treating pulmonary fibrosis, comprising the compound or a pharmaceutically acceptable salt thereof as an active ingredient.

Still another object of the present invention is to provide a method for preventing or treating pulmonary fibrosis using the pharmaceutical composition.

Still another object of the present invention is to provide a food composition for preventing or improving pulmonary fibrosis, comprising the compound or a pharmaceutically acceptable salt thereof as an active ingredient.

Still another object of the present invention is to provide a use of the compound or a pharmaceutically acceptable salt thereof; or a composition comprising the same in preventing, improving, or treating pulmonary fibrosis.

### [Advantageous Effects]

The composition comprising the compound of the present invention not only regulates the expression of phosphorylated proteins, such as p38, AKT, smad2, and smad7, but also inhibits the expression of pulmonary fibrosis marker genes and proteins by specifically inhibiting the SRF/MRTF signaling pathway, therefore, the composition comprising the compound as an active ingredient may be usefully applied as an effective treatment for pulmonary fibrosis.

### [Brief Description of the Drawing]

FIG. 1 confirms the cytotoxicity of sulforaphane-based synthetic compounds.
FIG. 2 shows the inhibitory effect of treatments with 16 compounds on the expression of pulmonary fibrosis marker proteins in lung fibroblasts induced by TGF-β1.
FIG. 3 shows the inhibitory effect of treatments with Compound 1 and Compound 2, which are sulforaphane-based synthetic compounds that exhibit inhibitory effects on pulmonary fibrosis, at different concentrations on the expression of pulmonary fibrosis marker proteins.
FIG. 4a to 4d show the observations on each of the results of cytotoxicity and morphological changes, analysis of pulmonary fibrosis marker protein expression, and analysis of pulmonary fibrosis marker gene expression in normal lung fibroblasts (MRC-5) and diseased lung fibroblasts (DHLF-IPF) according to the treatments with Compound 1 and Compound 2.
FIG. 5 shows the inhibitory effect of the treatments with Compound 1 and Compound 2 on cell migration in normal lung fibroblasts (MRC-5) and diseased lung fibroblasts (DHLF-IPF) through transwell migration analysis.
FIG. 6 shows the inhibitory effect of the treatments with Compound 1 and Compound 2 on cell migration in normal lung fibroblasts (MRC-5) and diseased lung fibroblasts (DHLF-IPF) through wound healing analysis.
FIG. 7 shows the analysis results of the expression of proteins related to signaling pathways involved in TGF-β1 induction in diseased lung fibroblasts (DHLF-IPF) according to the treatment with Compound 1.
FIG. 8 shows the analysis results of the expression of proteins related to signaling pathways involved in TGF-β1 induction in diseased lung fibroblasts (DHLF-IPF) according to the treatment with Compound 2.
FIGS. 9a and 9b show the analysis results of the changes in body weight and lung weight over time in bleomycin-induced pulmonary fibrosis animal models according to the treatments with Compound 1 and Compound 2.
FIGS. 10a and 10b show the analysis results of the changes in collagen content in lung tissue of bleomycin-induced pulmonary fibrosis animal models according to the treatments with Compound 1 and Compound 2.
FIGS. 11a and 11b show the analysis results of the histological changes in inflammation and fibrosis markers in lung tissue of bleomycin-induced pulmonary fibrosis animal models according to the treatments with Compound 1 and Compound 2.
FIGS. 12a and 12b show the histological analysis results of the expression of fibrosis-related protein markers in lung tissue of bleomycin-induced pulmonary fibrosis animal models according to the treatments with Compound 1 and Compound 2.
FIGS. 13a and 13b show the analysis results of the expression of genes related to fibrosis in lung tissue of bleomycin-induced pulmonary fibrosis animal models according to the treatments with Compound 1 and Compound 2.

### [Detailed Description of Preferred Embodiments]

The present invention will be described in detail as follows. Meanwhile, each description and embodiment disclosed in this invention may also be applied to other descriptions and embodiments. That is, all combinations of various elements disclosed in this invention fall within the scope of the present disclosure. Further, the scope of the present invention is not limited by the specific description described below.

An aspect of the present invention for achieving the above objects provides a compound represented by Formula I below or a pharmaceutically acceptable salt thereof. 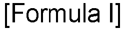 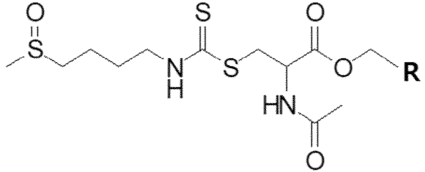 wherein, in Formula I above,
R is methyl or ethenyl.

The Formula I may specifically refer to Compound 1 or Compound 2 respectively represented by Formula 1 or Formula 2 below.

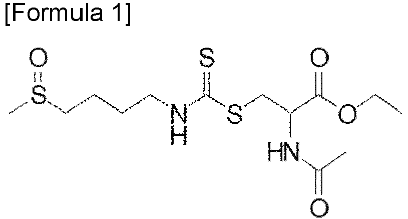

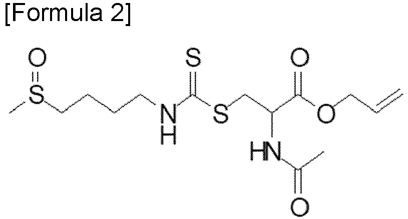

In the present invention, Formula 1 and Formula 2 are novel sulforaphane-based compounds having molecular weights of 368.5 and 380.5, respectively, which are more than twice that of sulforaphane, which has a molecular weight of 177.3. These compounds are characterized by the inhibition of the worsening or progression of pulmonary fibrosis.

The novel compound of the present invention may be chemically synthesized by a method known in the art and may exist in an unsolvated form as well as a solvated form. Further, the compound may exist in a crystalline or amorphous form, and all such physical forms are included within the scope of the present invention.

In the present invention, the term "pharmaceutically acceptable salt" refers to a salt conventionally used in the pharmaceutical industry. Examples may include inorganic salts made from metal ions such as calcium, potassium, sodium, magnesium, *etc.;* inorganic salts made from inorganic acid such as hydrochloric acid, nitric acid, phosphoric acid, bromic acid, iodic acid, perchloric acid, tartaric acid, sulfuric acid, *etc.;* organic salts made from organic acid such as acetic acid, trifluoroacetic acid, maleic acid, succinic acid, oxalic acid, benzoic acid, tartaric acid, fumaric acid, mandelic acid, propionic acid, citric acid, lactic acid, glycolic acid, gluconic acid, galacturonic acid, glutamic acid, glutaric acid, glucuronic acid, aspartic acid, ascorbic acid, carbonic acid, vanillic acid, hydroiodic acid, *etc.;* sulfonic acid salts made from methanesulfonic acid, ethanesulfonic acid, benzenesulfonic acid, p-toluenesulfonic acid, naphthalenesulfonic acid, *etc.;* amino acid salts made from glycine, arginine, lysine, and the like; and amine salts made from trimethylamine, triethylamine, ammonia, pyridine, picoline, *etc.* However, the types of salts referred to by the present invention are not limited to these listed examples.

Another aspect of the present invention for achieving the above objects provides a pharmaceutical composition for preventing or treating pulmonary fibrosis, comprising the novel compound or a pharmaceutically acceptable salt thereof as an active ingredient.

In the present invention, the term "pulmonary fibrosis" is a type of chronic interstitial lung disease also known as idiopathic pulmonary fibrosis (IPF), and refers to a disease in which lung tissue cells transform into fibrous cells, causing symptoms such as dyspnea, coughing, cyanosis, and clubbing. During tissue biopsies, honeycomb-like or atypical fibrous cell clusters are observed. Current treatments include the use of immunosuppressants including steroid-based therapy, interferon gamma, acetylcysteine, pirfenidone, nintedanib, bosentan, and the like, but there have been no reports of agents exhibiting specific therapeutic effects.

In the present invention, the pulmonary fibrosis may refer to one selected from the group consisting of chronic obstructive pulmonary disease combined pulmonary fibrosis (COPD combined pulmonary fibrosis), combined pulmonary fibrosis and emphyma, idiopathic pulmonary fibrosis (IPF), pulmonary fibrosis according to anticancer treatment, and pulmonary fibrosis caused by virus, specifically IPF, but is not limited thereto.

In the present invention, the term "prevention" refers to all actions that inhibit or delay the progression of pulmonary fibrosis by the administration of a pharmaceutical composition comprising the compound as an active ingredient.

In the present invention, the term "treatment" refers to all actions that improve or beneficially alter the symptoms of pulmonary fibrosis by the administration of a pharmaceutical composition comprising the compound as an active ingredient.

In the present invention, the term "pharmaceutical composition" may additionally include pharmaceutically acceptable carriers, excipients, or diluents conventionally used in the preparation of the pharmaceutical composition, and the carrier may include non-naturally occurring carriers. Specific examples of carriers, excipients, and diluents may include lactose, dextrose, sucrose, sorbitol, mannitol, xylitol, erythritol, maltitol, starch, acacia gum, alginate, gelatin, calcium phosphate, calcium silicate, cellulose, methylcellulose, microcrystalline cellulose, polyvinylpyrrolidone, water, methyl hydroxybenzoate, propyl hydroxybenzoate, talc, magnesium stearate, mineral oil, and the like, but are not limited thereto.

Additionally, the pharmaceutical composition may be in any form selected from the group consisting of tablets, pills, powders, granules, capsules, suspensions, solutions for internal use, emulsions, syrups, sterile aqueous solutions, non-aqueous solutions, suspensions, emulsions, freeze-dried preparations, and suppositories, and may be in various oral or parenteral forms. When formulated, the pharmaceutical composition is prepared using diluents or excipients such as fillers, extenders, binders, wetting agents, disintegrants, or surfactants that are conventionally used. Solid preparations for oral administration include tablets, pills, powders, granules, capsules, *etc.,* and the solid preparations may comprise one or more excipients, for example, starch, calcium carbonate, sucrose, lactose, gelatin, *etc.* In addition to simple excipients, lubricants such as magnesium stearate and talc, *etc.* may also be used. Liquid preparations for oral administration include suspensions, solutions for internal use, emulsions, and syrups, which may comprise commonly-used simple diluents such as water and liquid paraffin, as well as various excipients such as wetting agents, sweeteners, flavoring agents, and preservatives. For preparations for parenteral administration, sterile aqueous solutions, non-aqueous solutions, suspensions, emulsions, freeze-dried preparations, suppositories, and the like may be used. Non-aqueous solutions and suspensions may use propylene glycol, polyethylene glycol, vegetable oils such as olive oil, and injectable esters such as ethyl oleate. For bases for suppositories, witepsol, macrogol, tween 61, cacao butter, laurin butter, glycerogelatin, and the like may be used, but the additives to be added are not limited thereto.

The content of the compound in the pharmaceutical composition of the present invention may be appropriately adjusted according to the symptoms, progression, condition of the patient, and the like. For example, it may be 0.0001% to 99.9% by weight or 0.001% to 50% by weight based on the total weight of the composition, but is not limited to these ranges.

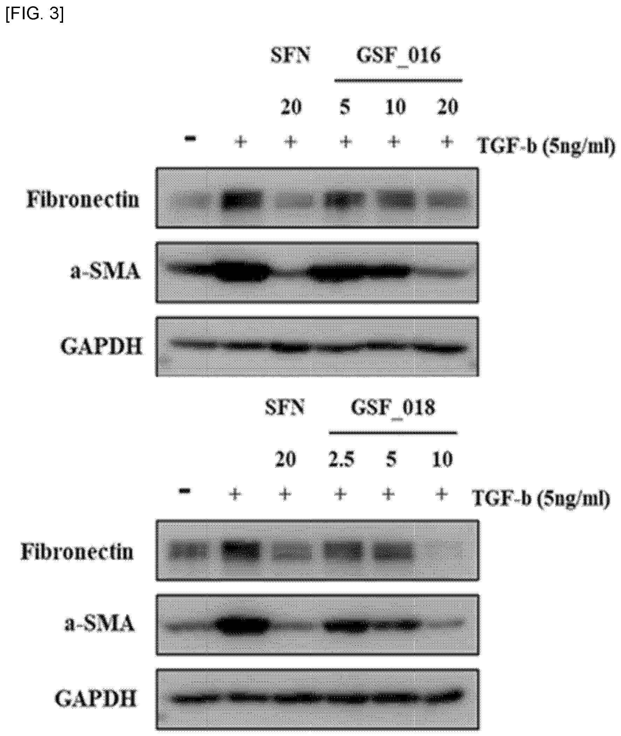

In one embodiment of the present invention, it was confirmed that the expression levels of fibronectin and α-SMA increased by TGF-β1 treatment were decreased in a dose-dependent manner by the treatments with Compound 1 and Compound 2, respectively, at different concentrations, and it was confirmed that the expression-reducing effect at a concentration of 20 µM or 10 µM for each compound was similar or superior to that of sulforaphane (FIG. 3).

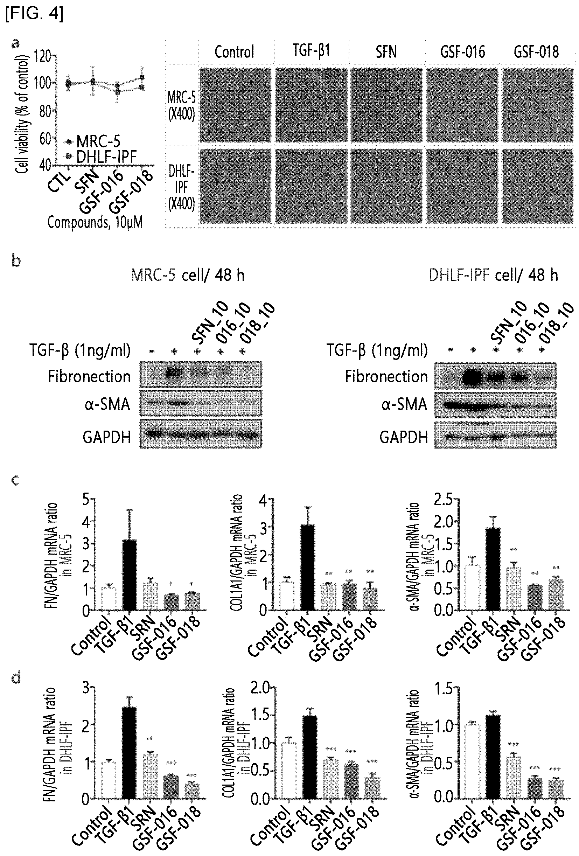

Further, in one embodiment of the present invention, MRC-5 (i.e., normal human lung fibroblasts) and diseased human lung fibroblasts-idiopathic pulmonary fibrosis (DHLF-IPF) (*i.e.*, diseased pulmonary fibroblasts) were treated with sulforaphane, Compound 1, and Compound 2, and the morphological changes of the cells were examined. As a result, it was confirmed that the inhibition of cell proliferation was more significant in the groups treated with Compound 1 and Compound 2 compared to that in the sulforaphane-treated group, and the cells showed a thin and elongated shape, which is similar to that of the control group, while not showing a densely packed structure (FIG. 4a).

Further, in one embodiment of the present invention, the effect on the expression of fibrosis-related proteins and genes in MRC-5 and DHLF-IPF fibroblasts was examined. As a result, it was confirmed that the expression levels of the proteins and genes induced by TGF-β1 were reduced by sulforaphane, Compound 1, and Compound 2 in both cells, and in particular, Compound 1 and Compound 2 showed the effect of significantly reducing the expression levels compared to sulforaphane (FIGS. 4b to 4d).

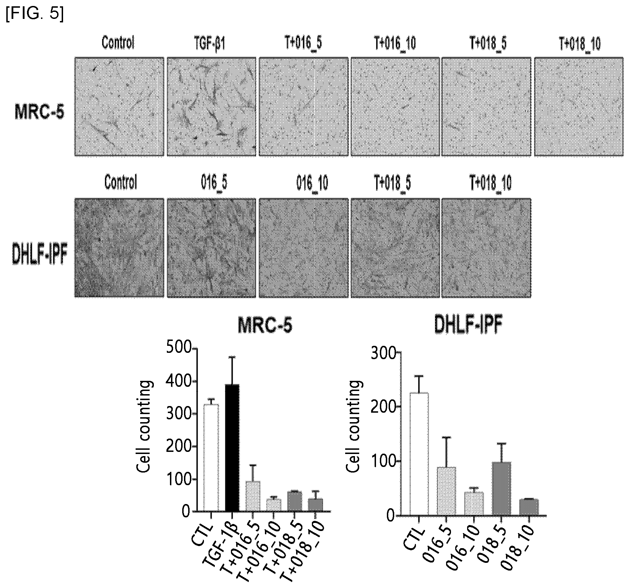

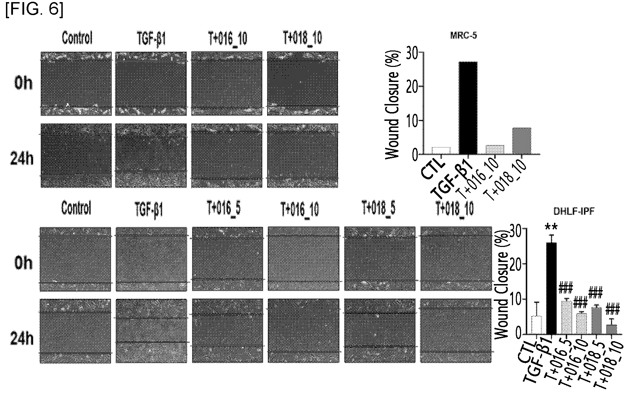

Further, in one embodiment of the present invention, the effect on cell migration in MRC-5 and DHLF-IPF was examined. As a result, it was confirmed that cell migration was increased by TGF-β1 treatment, and particularly in DHLF-IPF cells, the cell migration was clearly increased even without TGF-β1 induction but was significantly inhibited in a dose-dependent manner by the treatments with Compound 1 and Compound 2 (FIGS. 5 and 6).

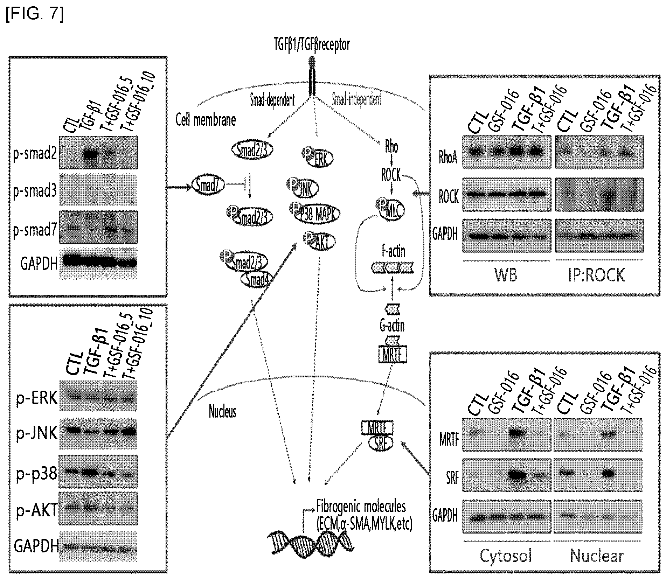

Further, in one embodiment of the present invention, the inhibitory effect of the treatment with Compound 1 on pulmonary fibrosis in DHLF-IPF was examined through expression analysis of various signaling pathway proteins associated with TGF-β1. As a result, it was confirmed that Compound 1 inhibited the phosphorylation of smad-2 while increasing the phosphorylation of smad-7, and significantly reducing the activity of p-p38 MAPK and p-AKT. Additionally, it was confirmed that Compound 1 inhibited the expression of ROCK in the Rho-ROCK signaling pathway and the expression of both nuclear MRTF and SRF in the MRTF-SRF signaling pathway (FIG. 7).

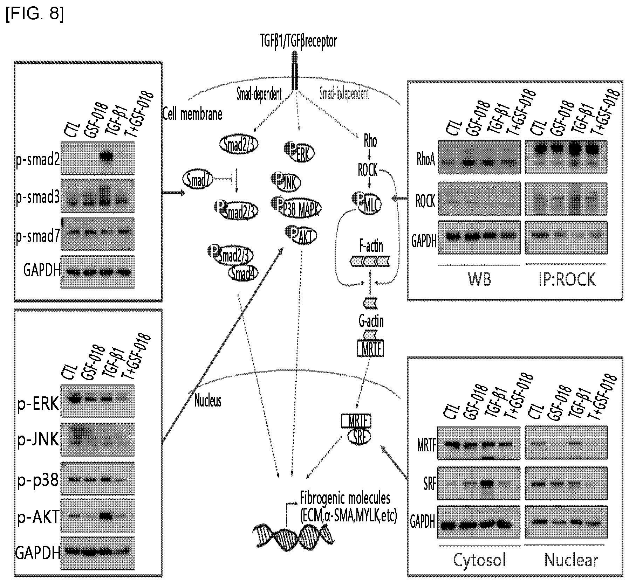

Further, in one embodiment of the present invention, the inhibitory effect of Compound 2 treatment on pulmonary fibrosis in DHLF-IPF was examined through expression analysis of various signaling pathway proteins associated with TGF-β1. As a result, it was confirmed that Compound 2 inhibited the phosphorylation of smad-2 and smad-3 while increasing the phosphorylation of smad-7, and significantly reduced the activity of three phosphorylated proteins (*i.e*.*,* p-ERK, p-JNK, and p-p38) in the MAPK signaling pathway, and significantly reduced the activity of p-AKT in the AKT signaling pathway. Additionally, it was confirmed that Compound 2 inhibited the expression of ROCK in the Rho-ROCK signaling pathway and the expression of both intranuclear MRTF and SRF in the MRTF-SRF signaling pathway (FIG. 8).

These results suggest that the compound of the present invention can be effectively used in preventing or treating pulmonary fibrosis.

Another aspect of the present invention for achieving the above objects provides a method for preventing or treating pulmonary fibrosis, comprising administering the pharmaceutical composition to a subject.

The pharmaceutical composition, pulmonary fibrosis, prevention, and treatment are as described above.

In the present invention, the term "subject" refers to humans and all animals including, mice, and livestock, in which pulmonary fibrosis has developed or may develop. Specifically, the subject may be mammals including cows, horses, sheep, pigs, goats, camels, antelopes, dogs, cats, *etc.,* as well as humans which need prevention or treatment of symptoms similar to those of the disease, but is not limited thereto.

Further, the subject may include humans or may exclude humans.

In the present invention, the term "administration" refers to introducing the composition of the present invention to a patient by an appropriate method. The route of administration of the composition may be administered through any general route as long as the target tissue can be reached by the same.

The pharmaceutical composition of the present invention may be administered in a pharmaceutically effective amount.

The term "pharmaceutically effective amount" refers to an amount sufficient to treat a disease at a reasonable benefit-risk ratio applicable to medical treatment, and the effective dose level may be determined based on factors including the type, age, and sex of the subject, severity of disease, activity of the drug, sensitivity to the drug, administration time, administration route and excretion rate, treatment period, and concomitant medications, and other factors well known in the medical field.

The pharmaceutical composition of the present invention may be administered as a single agent or in combination with other therapeutic agents, and be administered sequentially or simultaneously with conventional therapeutic agents. Further, the pharmaceutical composition may also be administered in single or multiple doses. It is important to administer an amount that can maximize the effect with minimal side effects considering all these factors, and this can easily be determined by those skilled in the art.

Further, the pharmaceutical composition may be administered orally or parenterally (for example, intravenously, subcutaneously, intraperitoneally, or topically). The dose differs according to the condition and weight of the patient, the severity of disease, form of drug, and route and time of administration, but it can be appropriately selected by those skilled in the art. In a specific example, the pharmaceutical composition may generally be administered once or several times a day, but the preferred dose may be appropriately selected by those skilled in the art according to the condition and weight of the subject, severity of disease, form of drug, and route and time of administration.

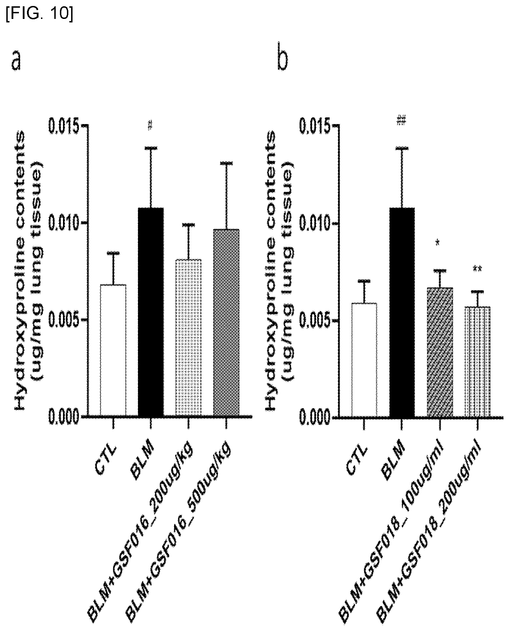

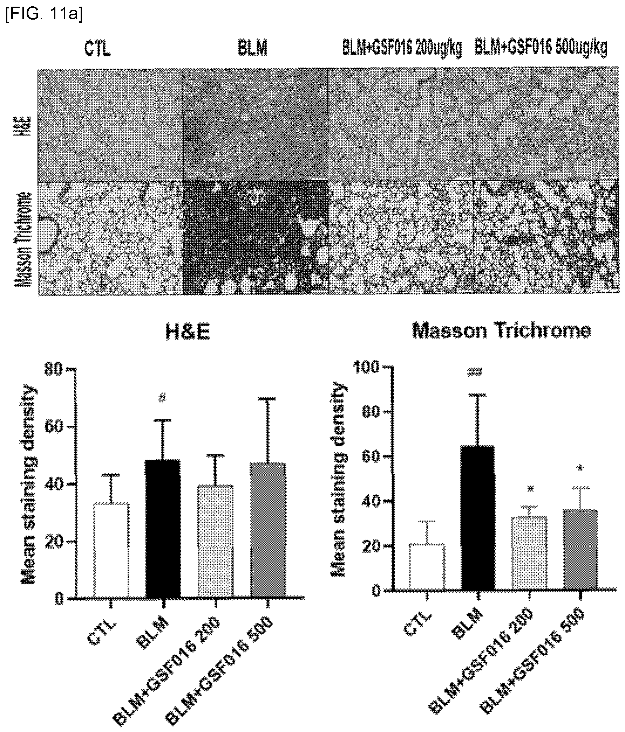

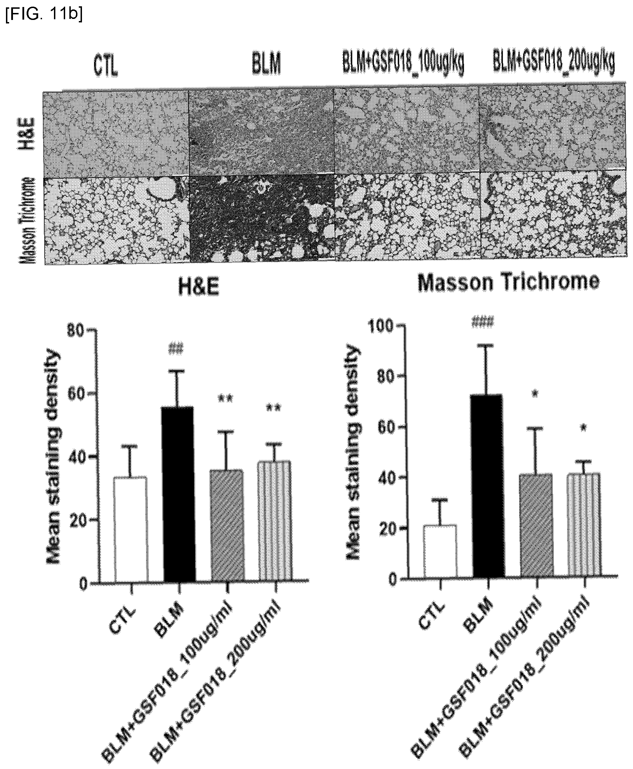

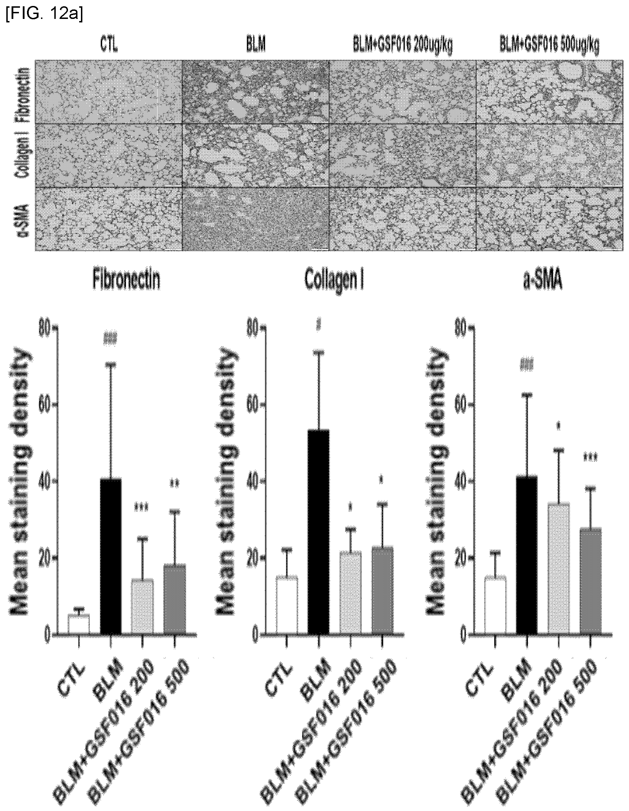

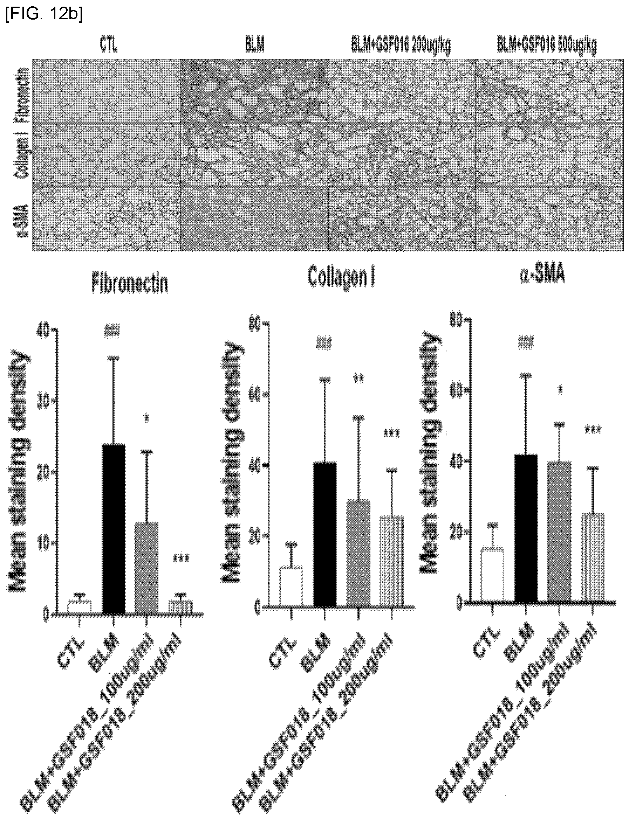

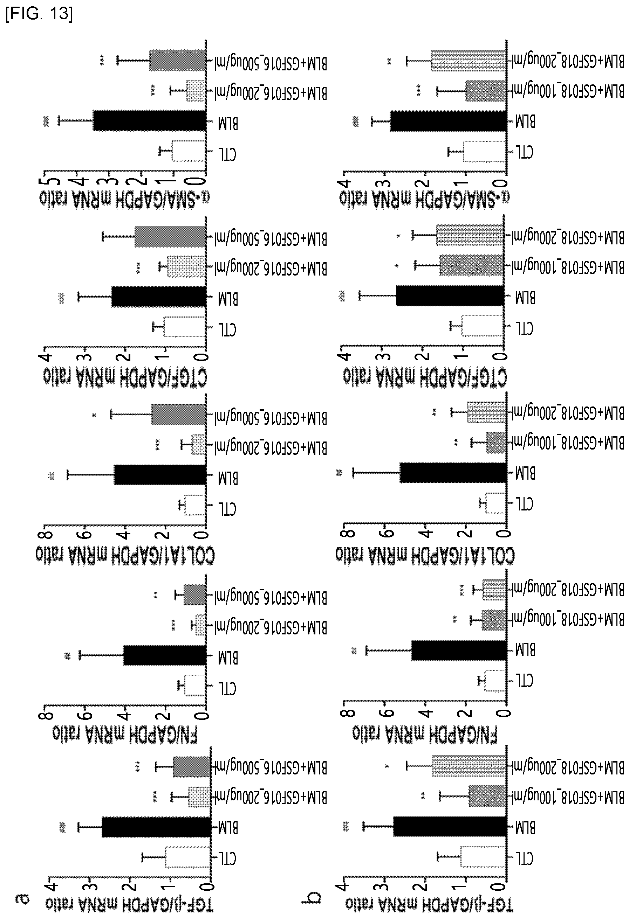

In one embodiment of the present invention, the inhibitory effect of the finally selected Compound 1 and Compound 2 on pulmonary fibrosis in pulmonary fibrosis animal models induced using bleomycin was examined. As a result, it was confirmed that the hydroxyproline content in lung tissues was significantly reduced in both compound-treated groups (FIG. 10), the degree of inflammation and fibrosis in lung tissues was decreased (FIG. 11), and the expression levels of proteins and genes related to fibrogenesis, such as α-SMA, collagen, and fibronectin, were also reduced (FIGS. 12 and 13).

Still another aspect of the present invention for achieving the above objects provides a food composition for preventing or improving pulmonary fibrosis, comprising the compound or a pharmaceutically acceptable salt thereof as an active ingredient.

The compound, pulmonary fibrosis, and prevention are as described above.

In the present invention, the term "improvement" refers to all actions that reduce parameters, such as the severity of symptoms, related to conditions that can be treated by the administration of a composition comprising the novel compound.

In the present invention, the term "food" may be meats, sausages, bread, chocolate, candies, snacks, confectionery, pizza, ramen, other noodles, gums, dairy products including ice creams, various soups, beverages, tea, drinks, alcoholic beverages, vitamin complexes, health functional foods, health foods, and the like, and comprises all foods in the conventional sense.

The term health functional food is synonymous with food for special health use (FoSHU) and refers to foods with high medical and therapeutic effects, processed to efficiently exhibit biological regulatory functions in addition to providing nutrition.

Herein, "function (functionality)" refers to the obtainment of useful effects for health purposes, such as regulating nutrients, physiological actions, *etc.* with regard to the structure and function of the human body. The health food refers to foods having an effect of actively maintaining or promoting health compared to general foods, and health supplement food refers to foods for health supplement purposes. Depending on the context, the terms health functional food, health food, and health supplement food may be used interchangeably. Specifically, the health functional food refers to foods which are prepared by adding the novel compound of the present invention to food materials, such as beverages, teas, spices, gums, and confectionery, or those prepared into capsules, powders, or suspensions, and may refer to those which may provide specific health effects upon their consumption.

The food of the present invention may be prepared by methods conventionally used in the art and by adding raw materials and ingredients conventionally added in the art.

Further, the food composition may be prepared into various types of formulations without limitation provided that the formulation is recognized as food.

Further, the food composition may further include a sitologically acceptable carrier, and the type of carrier is not particularly limited and may be any carrier conventionally used in the art.

Further, the food composition may include additional ingredients conventionally used in food compositions that can improve smell, taste, and appearance. Examples may include vitamins A, C, D, E, B1, B2, B6, B12, niacin, biotin, folate, pantothenic acid, and the like. Furthermore, it may also include minerals such as zinc (Zn), iron (Fe), calcium (Ca), magnesium (Mg), manganese (Mn), copper (Cu), and chromium (Cr); and amino acids such as lysine, tryptophan, cysteine, and valine.

Further, the food composition may include food additives such as preservatives (for example, potassium sorbate, sodium benzoate, salicylic acid, and sodium dehydroacetate), sterilizers (for example, bleaching powder and high performance bleaching powder, and sodium hypochlorite), antioxidants (for example, butylated hydroxyanisole (BHA) and butylated hydroxytoluene (BHT)), colorants (for example, tar pigment), color fixatives (for example, sodium nitrite and sodium nitrate), bleaching agents (for example, sodium sulfite), seasonings (for example, monosodium glutamate (MSG)), sweeteners (for example, dulcin, cyclamate, saccharin, and sodium), flavoring agents (for example, vanillin and lactones), leavening agents (for example, alum, and D-potassium hydrogen tartrate), reinforcing agents, emulsifiers, thickeners (pastes), coating agents, gum bases, anti-foaming agents, solvents, and improvers. These additives may be selected according to the type of food and used in appropriate amounts.

Still another aspect of the present invention for achieving the above objects provides a feed composition for preventing or improving pulmonary fibrosis, comprising the compound or a pharmaceutically acceptable salt thereof as an active ingredient.

The compound, pulmonary fibrosis, and prevention are as described above.

The term "feed" refers to any natural or artificial prescribed diet, meal, *etc.* for or suitable for consumption, ingestion, and digestion by animals, or components of the meal.

The type of feed is not particularly limited, and any feed that is conventionally used in the art may be used. Non-limiting examples of the feed may include plant-based feeds such as grains, nuts, food processing by-products, algae, fibers, pharmaceutical by-products, oils, starches, cakes, or grain by-products; and animal-based feeds such as proteins, minerals, inorganic substances, fats, minerals, oils, single-cell proteins, animal plankton, or food. These may be used alone or in combination of two or more.

Still another aspect of the present invention for achieving the above objects provides a use of the compound or a pharmaceutically acceptable salt thereof; or a composition comprising thereof in preventing, improving, or treating the composition. Further, it provides a use of the compound or a pharmaceutically acceptable salt thereof; or a composition comprising the same in preparing drug, food, or feed for preventing, improving, or treating pulmonary fibrosis.

The compound, pharmaceutically acceptable salt, pulmonary fibrosis, prevention, improvement, and treatment are as described above.

### [Mode for Carrying Out the Invention]

Hereinafter, the present invention will be described in detail by way of Examples. However, these Examples are given for illustrative purposes only, and the scope of the invention is not intended to be limited by these Examples.

### Synthesis Example 1: Synthesis of Novel Compounds

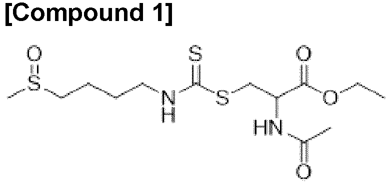

To an ethanol solution (2 mL) of N-acetyl-L-cysteine ethyl ester (CAS No. 59587-09-6, 150 mg, 0.78 mmol), 1 N NaOH was added to adjust the pH of the solution to 8. An ethanol solution (2 mL) of sulforaphane (CAS No. 4478-93-7, 50 mg, 0.28 mmol) was added thereto. The reaction mixture was stirred at room temperature under nitrogen for 4 hours. After evaporating the solvent, the residue was purified by reverse-phase column chromatography using a methanol solution containing 0.05% TFA to obtain Compound 1 (160 mg, yield 55%). ¹H NMR (600 MHz, CDCl₃) δ 9.09 (s, 1H), 7.02 (d, *J* = 7.4 Hz, 1H), 4.83 - 4.59 (m, 1H), 4.16 (q, *J* = 7.1 Hz, 3H), 3.80 - 3.68 (m, 3H), 3.67 - 3.49 (m, 1H), 3.07 - 2.81 (m, 1H), 2.78 - 2.67 (m, 1H), 2.56 (d, *J* = 1.2 Hz, 3H), 2.01 (d, *J* = 8.1 Hz, 1H), 1.95 (s, 3H), 1.81 (s, 1H), 1.24 (t, *J* = 7.1 Hz, 4H); ¹³C NMR (150 MHz, CDCl₃) δ 196.75, 170.67, 170.32, 61.99, 53.50, 53.08, 46.84, 38.47, 35.82, 26.94, 23.00, 20.19, 14.13; ESI-MS (positive mode): m/z calculated for C₁₃H₂₄O₄N₂NaS₃, [M +Na]⁺ = 391.53 found: 391.40.

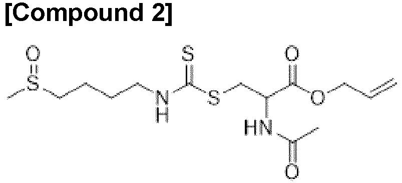

To an ethanol solution (2 mL) of Ac-Cys-OAllyl (CAS No. 616-91-1, 138 mg, 0.67 mmol), 1N NaOH was added to adjust the pH of the solution to 8. An ethanol solution (2 mL) of sulforaphane (CAS No. 4478-93-7, 100 mg, 0.56 mmol) was then added. The reaction mixture was stirred at room temperature under nitrogen for 10 hours. After evaporating the solvent, the residue was purified by reverse-phase column chromatography using a methanol solution of 0.05% TFA to obtain Compound 2 (112 mg, yield 52%). ¹H NMR (600 MHz, CD³OD) δ 6.09 - 5.90 (m, 1H), 5.42 - 5.31 (m, 1H), 5.31 - 5.11 (m, 1H), 4.76 - 4.68 (m, 1H), 4.68 - 4.59 (m, 2H), 3.95 (dd, *J =* 14.2 and 5.2 Hz, 1H), 3.77 - 3.71 (m, 1H), 3.52 (dd, *J* = 14.2 and 8.5 Hz, 1H), 2.96 - 2.76 (m, 3H), 2.65 (d, *J* = 10.4 Hz, 3H), 1.97 (s, 3H), 1.91 - 1.76 (m, 4H); ¹³C NMR (150 MHz, CD³OD) δ 197.75, 173.31, 171.59, 133.15, 118.59, 67.02, 54.26, 53.89, 45.48, 38.14, 34.74, 28.09, 22.36, 21.09; ESI-MS (positive mode): m/z calculated for C¹⁴H²⁴N²NaO⁴S³, [M +Na]⁺ = 403.08 found: 403.21.

### Example 1. Evaluation of cytotoxicity of sulforaphane-based synthetic compounds

### Cell Cultivation and Cytotoxicity Evaluation

The normal lung fibroblast MRC-5 cell line was obtained from the Korean Cell Line Bank. These cells were incubated at 37°C in a 5% CO₂ incubator using DMEM mixed with 10% FBS, 100 U/ml penicillin, and 100 µg/mL streptomycin. All cells used during the experiment were tested at a confluency of 80% to 90%.

To determine the optimal concentration of each compound synthesized from sulforaphane without toxicity, MRC-5 cells were seeded in 96-well plates at a density of 5 × 10³ cells/well in 100 µL of DMEM containing 10% FBS and cultured at 37°C in a 5% CO₂ incubator for 24 hours.

After the cultivation, the medium was removed, each compound was prepared at various concentrations to treat in triplicate for each group, and the cells were incubated at 37°C in a 5% CO₂ incubator for 24 hours. After incubation, 10 µL of 5 mg/mL MTT reagent was added to the medium comprising the test solution per well, and the plates were incubated in the dark at 37°C in a 5% CO₂ incubator for 2 to 4 hours. After completion of the incubation, the medium including the MTT reagent was removed, and 100 µL of DMSO (dimethyl sulfoxide) was added thereto to dissolve the MTT-formazan crystals. The absorbance the cells was measured at 570 nm to determine the cell viability compared to the control group. Cell viability (%) = (Absorbance of control group / Absorbance of experimental group) × 100

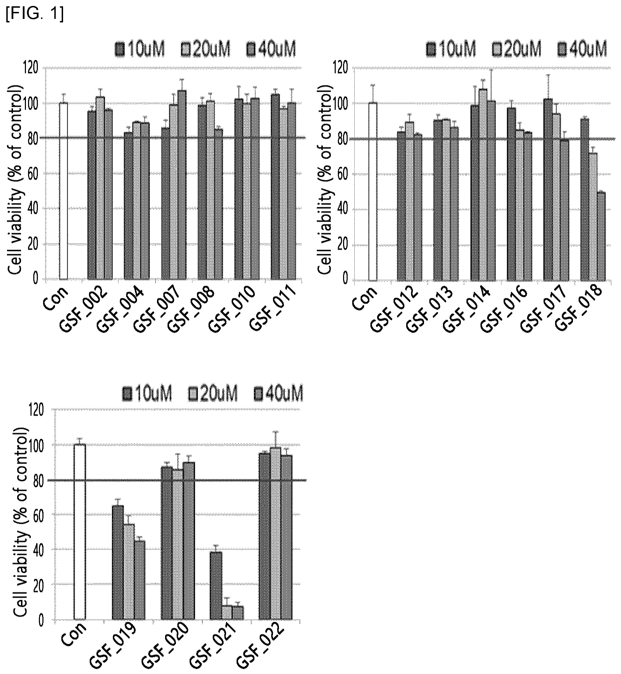

As a result, as shown in FIG. 1, a concentration that did not reduce cell viability below 80% compared to the untreated control group were to be used, and most compounds maintained cell viability of 80% or more even at 40 µM, and GSF-18 showed cell viability of 80% or more at 10 µM.

### Example 2. Analysis of fibrosis-related protein expression by sulforaphane- based synthetic compounds

Since the overexpression of extracellular matrix components such as fibronectin and α-SMA serves as an indicator of fibrotic changes in tissues, MRC-5 cells induced to undergo fibrosis by TGF-β1 were treated with each compound at different concentrations to compare the expression levels of fibronectin and α-SMA.

Specifically, to extract fibronectin and α-SMA proteins, the cells were washed once with cold PBS and then RIPA buffer (150 mM NaCl, 0.5% Triton X-100, 50 mM Tris-HCl, pH 7.4, 25 mM NaF, 20 mM EGTA), which was added with a protease inhibitor cocktail, was added thereto to lyse the cells. Then, the proteins were quantified using a BCA protein assay kit. All protein samples were subjected to electrophoresis at 10 µg on 8% to 10% PAGE gels and then transferred to 0.2 µm PVDF membranes (EMD Millipore, MA, USA). The membranes to which the protein was transferred were blocked with 5% BSA or 5% skim milk for 1 hour, and then primary antibodies were added thereto and incubated overnight at 4°C. The following day, the membranes were washed three times with PBST, and then HRP-conjugated secondary antibodies were added and reacted at room temperature for 1 hour. The PVDF membranes prepared through such procedures were smeared with ECL (Luminata^{™} Crescendo, EMD Millipore) and exposed to a fluorescence reader to confirm the level of protein expression.

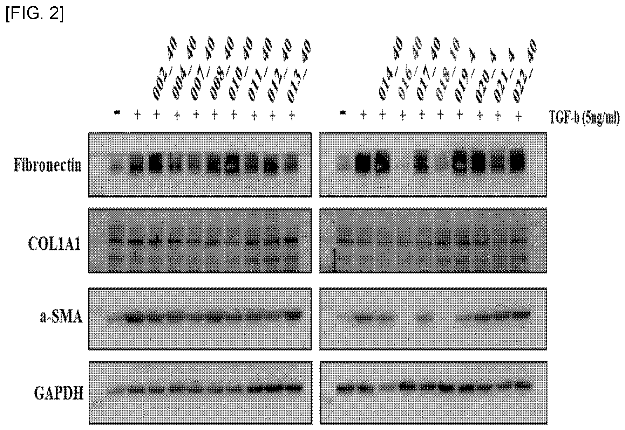

As a result, as shown in FIG. 2, the expression of fibronectin and α-SMA significantly increased when pulmonary fibrosis was induced by TGF-β1, and the expression levels of these two proteins were significantly decreased upon treatment with GSF-016 and GSF-018 among the sulforaphane compounds.

Therefore, these two compounds were named Compound 1 and Compound 2, respectively, and selected as candidate compounds for the treatment of fibrosis of the present invention.

### Example 3. Selection of sulforaphane prodrugs

### Example 3-1. Analysis of effect of candidate compounds on expression of fibrosis-related proteins

To compare the expression levels of fibronectin and α-SMA by concentration with treatment of sulforaphane (SFN), as well as Compound 1 and Compound 2 which markedly reduced the expression of these proteins increased by pulmonary fibrosis in Example 2, Western blotting was performed.

The specific experimental methods are the same as in Example 2, and sulforaphane was treated at a concentration of 20 µM, Compound 1 at 5 µM, 10 µM, and 20 µM, and Compound 2 at 2.5 µM, 5 µM, and 10 µM, respectively.

As a result, as shown in FIG. 3, it was confirmed that the expression of fibronectin and α-SMA decreased in a concentration-dependent manner by the treatments with Compound 1 and Compound 2 at various concentrations, and these compounds exhibited expression-reducing effects similar or superior to sulforaphane at a concentration of 20 µM or 10 µM.

### Example 3-2. Analysis of cytotoxicity and morphology of candidate compounds in two cell lines

The effects of the two candidate compounds of sulforaphane prodrugs on cytotoxicity and morphological changes in normal lung fibroblast MRC-5 cells and diseased lung fibroblast DHLF-IPF cells were examined. The diseased lung fibroblast DHLF-IPF cell line was purchased from Lonza and cultured in FGM^{™}-2 Fibroblast Growth Medium-2 BulletKit^{™} at 37°C in a 5% CO₂ incubator. All the cells used in the experiment were tested at a confluency of 80% to 90%.

Both cells were dispensed at 5 × 10³ cells/well in 100 µL each in a 96-well plate and cultured at 37°C in a 5% CO₂ incubator for 24 hours. Then, the medium was removed, and each compound prepared at a concentration of 10 µM in triplicate per group, followed by incubation at 37°C in a 5% CO₂ incubator for 24 hours.

The detailed experimental methods for cytotoxicity are the same as in Example 1.

Additionally, the changes in cell morphology of two lung fibroblasts induced by TGF-β1 according to the treatments with Compound 1 and Compound 2 were examined with the naked eye through a microscope.

As a result, as shown in FIG. 4a, both compounds exhibited cell viability of 80% or more at a concentration of 10 µM in both cell lines. Upon examination of the morphological changes in the two cell lines, it could be seen that the proliferation of the cells in the TGF-β1-treated group was more activated compared to those with a long and thin shape in the control group, inducing the cells to be interwoven and densely packed like fibrous tissues. In contrast, it was confirmed that the two compounds inhibited cell proliferation more effectively than the sulforaphane-treated group, thereby showing a long and thin cell shape similar to the control group and not appearing as dense as the fibrous tissue.

### Example 3-3: Analysis of effect of candidate compounds on expression of fibrosis-related protein in two cell lines

To compare the changes in fibrosis-related protein expression by treating normal MRC-5 and diseased DHLF-IPF cell lines induced with pulmonary fibrosis by TGF-β1 with SFN and the two candidate compounds, Western blotting was performed.

The specific experimental methods are the same as in Example 2. TGF-β1 was treated at 1 ng/mL to induce fibrosis, and sulforaphane and the two compounds each were treated at a concentration of 10 µM.

As a result, as shown in FIG. 4b, a significant decrease in the expression levels of fibronectin and α-SMA protein was confirmed by the treatment of the two compounds compared to sulforaphane.

### Example 3-4: Analysis of effect of candidate compounds on expression of fibrosis-related gene in two cell lines

To confirm whether SFN and the two compounds, which effectively inhibited the expression of fibrosis-related proteins in normal MRC-5 and diseased DHLF-IPF cells induced with fibrosis by TGF-β1, also have the same effect at the gene level, the gene expression of FN, COL1A1, and α-SMA was confirmed using qRT-PCR analysis.

### RNA Extraction from Cells

MRC-5 and DHLF-IPF cells were each cultured in growth medium, and the medium was removed. The experimental groups (SFN, GSF-016, and GSF-018) were pretreated with serum-free medium at a concentration of 10 µM for 1 hour, added with 1 ng/mL TGF-β1 to induce fibrosis, and incubated at 37°C in a 5% CO₂ incubator for 48 hours. The medium was then removed, and the cells were washed once with PBS. Intracellular total RNA was extracted from the cultured cells using TRIzol reagent (TaKaRa Bio Inc., Japan) for gene expression analysis. 1 mL of TRIzol reagent was added to lyse the cells and denature the tissue. The lysate was transferred to each 1.5 mL tube, and 200 µL of chloroform was added thereto, followed by vortexing for 20 seconds to ensure complete mixing. After incubating at room temperature for 15 minutes, the mixture was centrifuged at 14,000 rpm for 20 minutes to obtain the supernatant. After adding an equal volume of isopropyl alcohol, the tubes were inverted and incubated at room temperature for 10 minutes. The sample was centrifuged at 14,000 rpm for 15 minutes to obtain an RNA pellet, which was washed by centrifugation with 70% ethanol for use on RNA at 14,000 rpm for 5 minutes, and dried for 5 minutes. The dried RNA sample was added with 20 µL of distilled water treated with 0.1% diethyl pyrocarbonate (DEPC) to dissolve the pallet and the resultant was used as a sample for cDNA synthesis. 1 µL of the same was used to measure the RNA concentration and purity at O.D. 260/280 nm using a Nanodrop.

### cDNA Synthesis

Single-stranded cDNA synthesis was performed by mixing 1 µg of the extracted total RNA with 1 µL of oligo-d(T) primer (100 pmol) and 10 mM dNTP (TaKaRa Bio Inc., Japan), reacting at 65°C for 5 minutes, followed by rapid cooling. To this template, 4 µL of 5x RT buffer, 0.5 µL of RNA inhibitor, and 100 units of RTase (TaKaRa Bio Inc., Japan) were added, and the total volume was adjusted to 20 µL using DEPC-treated distilled water. The sample was synthesized at 25°C for 5 minutes, 42°C for 1 hour, and then reacted at 72°C for 15 minutes to inactivate the reverse transcriptase and terminate the reaction.

### RT-PCR

Each gene expression level was measured using real-time PCR. To 5 µL of the extracted cDNA of each sample, 10 µL of 2x SYBR Green MasterMix (TaKaRa Bio Inc., Japan) and 1 µL of 10 pmol (forward, reverse) primers for each gene were added to a final volume of 20 µL using distilled water. The PCR was performed at 95°C for 10 minutes (for 30 seconds from the 2^{nd} cycle), at 60°C for 30 seconds, and 30 seconds at 72°C and 40 cycles followed. The specificity of the amplified product was confirmed through melting curve analysis, and the target genes were quantified and compared using GAPDH as a control gene.

As shown in FIGS. 4c and 4d, the increased gene expression levels of FN, COL1A1, and α-SMA in both cell lines were reduced by treatment with SFN and the two compounds. In particular, it was confirmed that the groups treated with the two compounds showed a more significant decrease in expression levels compared to the SFN treatment group.

Based on these results, Compound 1 and Compound 2 were selected as the final compounds for the treatment of pulmonary fibrosis of the present invention among the sulforaphane-based synthetic compounds.

### Example 4. Cell migration

In accordance with the characteristics of myofibroblasts, in which the increased expression of proteins related to fibrogenesis leads to an increase of cell migration and invasion, migration and wound healing assays were conducted to examine the effects of the final selected Compound 1 and Compound 2 on cell migration using normal and diseased fibroblasts.

### Example 4-1. Transwell migration assay

A 24-well culture plate was prepared with 500 µL of medium including FBS, and an 8 µm pore size insert was placed on top. Inside the insert, 300 µL of medium without FBS was added, and MRC-5 and DHLF-IPF cells were seeded at a density of 1 × 10⁵ cells. The cells were incubated at 37°C in a 5% CO₂ incubator for 24 hours. After incubation, the medium inside the 8 µm pore polycarbonate membrane insert was carefully removed, washed with DPBS, and fixed in methanol for 5 minutes. The cells were then stained with Mayer's Haematoxylin for 8 minutes after washing twice with triple-distilled water. The stained cells were washed with DPBS, gently wiped with a cotton swab inside the insert, and the membrane was cut out and mounted on a slide. The migrated cells were counted under a microscope at 200x magnification.

As shown in FIG. 5, it was confirmed that the migration of normal MRC-5 cells increased with TGF-β1 treatment, and was significantly inhibited in a dose-dependent manner by Compound 1 and Compound 2. Similarly, in diseased DHLF-IPF cells, inherent high migration was observed, and it was significantly inhibited in a dose-dependent manner by Compound 1 and Compound 2, similar to MRC-5 cells.

### Example 4-2. Wound healing assay

The effects of Compound 1 and Compound 2, selected in Example 3, on cell migration (wound healing) in MRC-5 and DHLF-IPF cells were examined.

Specifically, cells (1 × 10⁵ cells/well) were seeded in a 60 mm dish to achieve approximately 90% confluency and cultured for 24 hours. Subsequently, a straight line was scratched on the bottom with a 200 µL pipette tip, and the detached cells were removed by washing once with Dulbecco's Phosphate-Buffered Saline (DPBS). The wound was photographed under a microscope, and treatments with TGF-β1 alone, TGF-β1+SFN, TGF-β1+GSF-016, and TGF-β1+GSF-018 were applied and cultured for approximately 24 hours, after which the wound was re-photographed. The images taken at 24 hours of culture were analyzed using the Image J (Fiji package) software to measure the distance between cells, and the relative migration was expressed as a percentage (%) compared to the control group.

As shown in FIG. 6, the migration of both MRC-5 and DHLF-IPF cells increased with TGF-β1 treatment, resulting in a narrower wound compared to the control group, while all compound-treated groups showed significant dose-dependent inhibition of migration.

### Example 5. Analysis of expression of proteins related to TGF-β1 signaling pathway

The TGF-β1 signaling pathway can be activated through both Smad-dependent and Smad-independent pathways. The TGF-β/Smad signaling, which is a Smad-dependent pathway, is a crucial pathway that regulates the synthesis of extracellular matrix components. The Smad-independent (TGF-β/non-Smad) pathway is influenced by PI3K/AKT/mTOR and MAPK (JNK, ERK, p38), which are known to be involved in cell proliferation and growth. Based on this mechanism, the protein expression related to the TGF-β1 signaling pathway was comparatively analyzed using the same Western blotting method conducted in Example 2.

As shown in FIG. 7, Compound 1 inhibited the phosphorylation of Smad-2 and increased the phosphorylation of Smad-7, significantly reducing the activity of p-p38 and MAPK. Additionally, the influence of MRTF/SRF via the Rho/Rock pathway was confirmed. The expression level of Rock protein, which binds to Rho activated by TGF-β1 treatment, was increased, and the expression levels of MRT/SRF in the cytoplasm and nucleus were also increased. However, the increased protein expression levels were significantly reduced by the treatment with Compound 1.

Furthermore, as shown in FIG. 8, Compound 2 inhibited the phosphorylation of Smad-2 and Smad-3 and increased the phosphorylation of Smad-7, reducing the activity of the three MAPKs: p-ERK, p-JNK, and p-p38. Additionally, the influence of MRTF/SRF via the Rho/Rock pathway was confirmed. The expression level of Rock protein, which binds to Rho activated by TGF-β1 treatment, was increased, and the expression levels of MRT/SRF in the cytoplasm and nucleus were also increased. However, the increased protein expression was significantly reduced by the treatment with Compound 2.

### Example 6. Inhibitory effect on pulmonary fibrosis in animal models

### Example 6-1. Analysis of body weight and lung weight changes in pulmonary fibrosis animal models treated with Compound 1 and Compound 2

The inhibitory effects of the finally selected Compound 1 and Compound 2 on pulmonary fibrosis were examined in bleomycin (BLM)-induced pulmonary fibrosis animal models.

Specifically, 7-week-old C57BL/6N mice were exposed to BLM (3-5 units/kg) via the airway. The compounds were administered orally at doses of 200 µg/kg and 500 µg/kg for Compound 1, and 100 µg/kg and 200 µg/kg for Compound 2, three times a week for three weeks starting the day after BLM exposure. Body weight gain was periodically measured during the experimental period, and lung weight was measured at the end of the experiment.

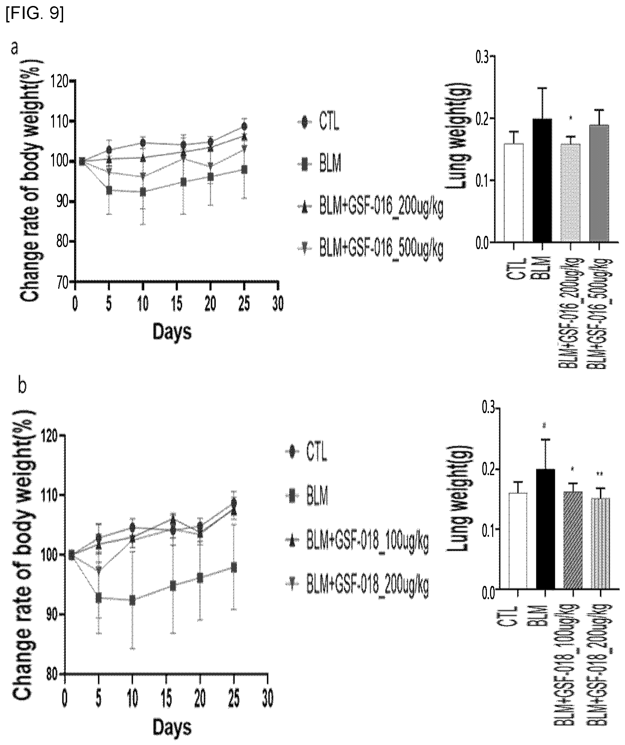

As shown in FIG. 9a, the body weight gain during the experimental period indicated that the normal group (CTL) was steadily increased, while the BLM group and the BLM+GSF-016_500 µg/kg group was initially decreased and then gradually increased. In contrast, the BLM+GSF-016_200 µg/kg group showed a steady increase. Lung weight measurements revealed that the BLM group had the highest lung weight, while the normal group and the BLM+GSF-016_200 µg/kg group had similar levels.

For Compound 2, as shown in FIG. 9b, the BLM group showed a significant decrease in body weight, which then gradually increased, but remained significantly lower compared to the normal and GSF-018 groups. In contrast, the BLM+GSF-018_100 µg/kg and BLM+GSF-018_200 µg/kg groups showed a steady increase in body weight, approaching levels similar to the normal group. Lung weight measurements indicated that the BLM group had significantly higher lung weight, while the normal group, BLM+GSF-018_100 µg/kg group, and BLM+GSF-018_200 µg/kg group had similarly lower levels, showing significant differences compared to the BLM group.

### Example 6-2. Quantitative analysis of hydroxyproline in lung tissue

To determine another indicator of fibrosis, the hydroxyproline content, which indirectly shows collagen content in lung tissue, was measured using a hydroxyproline colorimetric assay kit through ELISA analysis.

Specifically, homogenized lung tissue was placed in 1.5 mL tubes in similar amounts to prevent thawing, and 100 µL of 12 M hydrochloric acid was added for hydrolysis at 120°C for 3 hours. After centrifugation at 10,000 × g for 5 minutes, the supernatant was transferred to a new tube, and 10 µL of the sample was transferred to a 96-well plate and evaporated at 60°C. Subsequently, 100 µL of chloramine T reagent/oxidation buffer mixture was added to each well, followed by 100 µL of para-dimethylaminobenzaldehyde reagent, and reacted at 60°C for 90 minutes. Absorbance was measured at 540 nm, and the content was determined using a standard calibration curve prepared in the same manner.

As shown in FIGS. 10a and 10b, Compound 1 significantly increased hydroxyproline content in the BLM group, which was reduced in the BLM+GSF-016_200 µg/kg group. For Compound 2, both BLM+GSF-018_100 µg/kg and BLM+GSF-018_200 µg/kg groups showed significant reductions in hydroxyproline content compared to the BLM group.

### Example 6-3. Histological evaluation of the lung - 1

To observe changes in lung tissue, Hematoxylin and Eosin (H&E) staining and Masson-trichrome staining were performed on lung tissue samples to assess the degree of inflammation and fibrosis.

Specifically, the excised lung tissue was embedded in paraffin and sectioned into 4 µm slices using a microtome. These tissue sections were used for H&E, MT stain, and Immunohistochemistry (IHC). Each experiment was conducted by deparaffinizing and rehydrating the sections using xylene and ethanol. For IHC, the LSAB2 system HRP (DAKO, Carpinteria, CA, USA) kit was used. Antibody retrieval was performed in 0.1 mM citric acid (pH 6), and the experiment was conducted according to the provided instructions. The sections were blocked with blocking buffer at room temperature for 1 hour, then incubated overnight at 4°C with primary antibodies (fibronectin, collagen 1, α-SMA). After blocking with secondary antibodies at room temperature for 1 hour, the Streptavidin-HRP system was used for 10 minutes, followed by exposure to DAB solution, hematoxylin staining, dehydration, clearing, and mounting with Canada balsam. The stained slides were examined under an optical microscope (BK51, Olympus, Japan). The staining results were quantified using the Image J program.

As shown in FIGS. 11a and 11b, the normal group exhibited minimal inflammatory response, while the BLM group showed a significant increase in inflammation due to lymphocytes, neutrophils, and macrophages, with severe fibrosis and structural collapse of the lung tissue.

However, the group treated with Compound 1 showed a significant reduction in inflammation regardless of concentration, and the degree of fibrosis in the lung tissue was significantly reduced in the BLM+GSF-016_200 µg/kg and BLM+GSF-016_500 µg/kg groups compared to the BLM group.

Similarly, the group treated with Compound 2 showed a significant reduction in inflammation at both concentrations, and the degree of fibrosis in the lung tissue was significantly reduced in the BLM+GSF-018_100 µg/kg and BLM+GSF-018_200 µg/kg groups compared to the BLM group.

### Example 6-4. Histological evaluation of lung-2

To examine the key factors involved in fibrosis, immunohistochemistry (IHC) was performed using the same method as in Example 6-3.

As shown in FIGS. 12a and 12b, Compound 1 showed increased expression of α-SMA, collagen, and fibronectin in the BLM group compared to the normal group, similar to the pattern observed in IPF cell lines. The BLM+GSF-016 group showed a significant reduction in these proteins, with the best inhibitory effect on weight recovery and related protein expression observed at the lower concentration of 200 µg/kg.

Compound 2 also showed increased expression of fibronectin, collagen, and α-SMA in the BLM group compared to the normal group, with a significant reduction in the BLM+GSF-018 group in a dose-dependent manner.

### Example 6-5. Analysis of fibrosis-related gene expression in lung tissue

To confirm whether the expression of various fibrosis-related genes, including fibronectin, collagen, and α-SMA, showed the same pattern as the histological observations in the previous examples, qRT-PCR analysis was performed.

Specifically, to extract RNA from lung tissue, approximately 100 mg of pulverized lung tissue was dispensed into 1.5 mL tubes, and total RNA was extracted using TRIzol reagent (TaKaRa Bio Inc., Japan). After adding 1 mL of TRIzol reagent to denature the tissue, the samples were transferred to 1.5 ml tubes, and 200 µl of chloroform was added, followed by vortexing for 20 seconds to mix thoroughly. After reacting at room temperature for 15 minutes, the samples were centrifuged at 14,000 rpm for 20 minutes to obtain the supernatant. An equal volume of isopropyl alcohol was added, inverted, and allowed to stand at room temperature for 10 minutes. The samples were centrifuged at 14,000 rpm for 15 minutes to obtain RNA pellets, washed with 70% ethanol at 14,000 rpm for 5 minutes, and dried for 5 minutes. The dried RNA samples were dissolved in 20 µL of DEPC-treated distilled water and used for cDNA synthesis. The RNA concentration and purity were measured at OD 260/280 nm using a Nanodrop.

As shown in FIGS. 13a and 13b, Compound 1 significantly increased the expression of five genes (FN, COL1A1, α-SMA, TGF-β, and CTGF) in the BLM group compared to the normal group, with a significant reduction observed with Compound 1 treatment. Similarly, Compound 2 also significantly increased the expression of these five genes in the BLM group compared to the normal group, and a significant reduction was observed with Compound 2 treatment.

Those of ordinary skill in the art will recognize that the present disclosure may be embodied in other specific forms without departing from its spirit or essential characteristics. The described embodiments are to be considered in all respects only as illustrative and not restrictive. The scope of the present disclosure is therefore indicated by the appended claims rather than by the foregoing description. All changes which come within the meaning and range of equivalency of the claims are to be embraced within the scope of the present disclosure.

## Claims

1. A compound represented by the following Formula I or a pharmaceutically acceptable salt thereof: 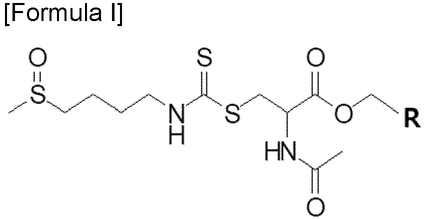 wherein, in Formula I above,
R is methyl or ethenyl.

2. The compound of claim 1, wherein the compound inhibits the worsening or progression of pulmonary fibrosis.

3. The compound of claim 1, wherein the compound is a sulforaphane-based compound.

4. A pharmaceutical composition for preventing or treating pulmonary fibrosis, comprising the compound of claim 1 or a pharmaceutically acceptable salt thereof as an active ingredient.

5. The pharmaceutical composition of claim 4, wherein the composition further comprises a pharmaceutically acceptable carrier or excipient.

6. The pharmaceutical composition of claim 4, wherein the pulmonary fibrosis is one or more selected from the group consisting of chronic obstructive pulmonary disease combined pulmonary fibrosis (COPD combined pulmonary fibrosis), combined pulmonary fibrosis and emphysema, idiopathic pulmonary fibrosis (IPF), pulmonary fibrosis caused by cancer treatment, and pulmonary fibrosis caused by viruses.

7. The pharmaceutical composition of claim 4, wherein the compound inhibits the expression of pulmonary fibrosis marker gene and protein by regulating the expression of a phosphorylated protein and the MRTF/SRF signaling pathway.

8. The pharmaceutical composition of claim 7, wherein the phosphorylated protein is p38, AKT, smad2, or smad7.

9. The pharmaceutical composition of claim 7, wherein the regulation of the MRTF/SRF signaling pathway reduces the increased expression of MRTF/SRF in the cytoplasm and nucleus caused by the increased expression of Rock protein that binds to Rho activated by TFG-β1 treatment.

10. A method for preventing or treating pulmonary fibrosis, comprising administering the pharmaceutical composition of any one of claims 4 to 9 to a subject.

11. A food composition for preventing or improving pulmonary fibrosis, comprising the compound of claim 1 or a pharmaceutically acceptable salt thereof as an active ingredient.
